# EUROPEAN PATENT APPLICATION

(11) **EP 4 009 050 A1**
(43) Date of publication of application: **08.06.2022**
(21) Application number: 21461629.4
(22) Date of filing: 03.12.2021
(51) Int. Cl.: G01N 33/569, A61B 5/053, G01N 27/02, A61K 39/215, A61B 5/145, A61B 5/08

(54) **BIOLOGICAL SENSOR FOR DETECTION OF PROTEINS OF VIRUSES AND DEVICE FOR DETECTION OF PROTEINS OF VIRUSES**

(30) Priority: 07.12.2020 PL 43625720
(71) Applicant: Advanced Diagnostic Equipment Spolka z Ograniczona Odpowiedzialnosica, 31-339 Krakow (PL); Centrum Badan I Rozwoju Technologii DLA Przeymyslu Spolka Akcyjna, 00-645 Waraszawa (PL)
(72) Inventor: Putynkowski, Grzegorz, 00-645 Warszawa (PL); Rydosz, Artur, 31-339 Krakow (PL); Marszalek, Konstatnty, 31-339 Krakow (PL)
(74) Representative: Bury & Bury

(57) **Abstract**

A biological sensor for detection of proteins of viruses comprises electrodes connected to an impedance meter, is provided with a common electrode (**E0**), a first electrode (E1), and a second electrode (E2), wherein those electrodes (**E0**, **E1**, **E2**) are provided in a common substrate so that they have a first contact area (**O1**) covered with a first substance comprising antibodies binding proteins of a first virus, in which in the said first contact area (**O1**), a distance (**W1**) between an edge of the common electrode (**E0**) and an edge of the first electrode (**E1**) is falling within a range of 8 to 12 pm and a height of the electrodes is falling within a range of 50 to 150 nm, and those electrodes (**E0**, **E1, E2**) have a second contact area (**O2**) covered with a second substance comprising antibodies binding proteins of the second substance, in which in the said second contact area (**O2**), a distance (**W2**) between the common electrode (**E0**) and the second electrode (**E2**) is falling within a range of 18 µm to 24 µm, whereas a height of electrodes above the substrate is falling within a range of 50 to 450 nm, wherein the common electrode (**E0**) and the first electrode (**E1**) are connected to the bridge impedance meter and the common electrode (**E0**) and the second electrode (**E2**) are connected to the bridge impedance meter.

A device for detection of proteins of viruses, according to the invention comprises a measurement chamber and a biological sensor, suitable for detection of proteins of viruses according to the invention.

## Description

The invention concerns a biological sensor suitable for detecting of proteins of viruses and a device for detection of proteins of viruses, in particular of proteins of viruses from a group of coronaviruses, in particular SARS-CoV-2, and proteins of influenza viruses.

A biological sensor is known from European patent description no. EP1996734B1, suitable for detecting of viruses, comprising electrodes covered with a layer of a substance suitable for a selective attachment of viruses, for example by N-hydroxysuccinimide monoactive ester (abbreviated NHS), connected to a system of resistance measurement, in particular impedance. Viruses are detected in such way, that firstly subjected to the measurement is the impedance before a contact of the electrode with a substance which potentially comprises viruses and then after contact with this substance and on a basis of the change in impedance measurement result it is established, whether or not detection of the virus has taken place. Virus particles can be obtained from the libraries presented on phages to detect a wide range of targets, including for example DNA, RNA, small particles and proteins or polypeptides. In other embodiment, the virus particles are electrostatically bonded with a substrate between a pair of elongated electrodes placed on the substrate.

Infection with the virus SARS-CoV-2 (causing the disease COVID-19) and the influenza virus gives similar symptoms among patients. However, due to other infectivity of those viruses and other course of the disease and complications, patients infected with SARS-CoV-2 and influenza require substantially different treatment. There is a lack of a method allowing for fast and unambiguous diagnosis of a patient with symptoms of COVID-19 and/or influenza. The only solution is keeping the patient under observation and carrying out tests. It is very troublesome due to the fact, that patients infected with SARS-CoV-2 should be isolated due to a risk of further infection of non-infected people and at the same time they should not be additionally exposed to influenza virus as well as other pathogens. Carrying out separate tests involves a risk of confusion. Because of commonness of both diseases, the risk of confusing the samples during their forwarding is statistically significant. Therefore, there is a need to provide a device and a method of unambiguous diagnosis of influenza and COVID-19 in one examination, on a basis of a single sample. Similarity of those viruses makes difficult to provide a selectivity of the device and the method allowing for their differentiation.

A biological sensor for detection of proteins of viruses comprising electrodes connected to an impedance meter, according to the invention is provided with a common electrode, a first electrode, and a second electrode, wherein those electrodes are provided in a common substrate so that they have a first contact area covered with a first substance comprising antibodies binding proteins of a first virus, in which in the said first contact area, a distance between an edge of the common electrode and an edge of the first electrode is falling within a range of 8 to 12 µm and a height of the electrodes is falling within a range of 50 to 150 nm. The electrodes have also a second contact area covered with a second substance comprising antibodies binding proteins of a second virus, in which in the said second contact area, a distance between the common electrode and the second electrode is falling within a range of 18 µm to 24 µm, whereas a height of electrodes above the substrate is falling within a range of 50 to 450 nm. The common electrode and the first electrode are connected to the bridge impedance meter. Also the common electrode and the second electrode are connected to the bridge impedance meter.

Advantageously, the common electrode, the first electrode and the second electrode have finger-like protrusions in the contact areas.

Advantageously, the substance comprising antibodies binding proteins of the first virus is a substance attaching specific proteins of viruses from a group of coronaviruses, advantageously specific proteins of SARS-CoV-2 virus.

Depending on parameters of technological process, the electrodes can have different heights. The height of the electrode above the substrate can change on a surface of the substrate. The thinner the electrodes, the more difficult it is to control their shape. Using of the electrodes of lower height should advantageously meet a condition, that the antibodies placed substantially perpendicular to the substrate have arms protruding above metallization of the electrodes in the contact area.

Providing the distance between the edges of the electrodes in the first contact area falling within a range of 8 to 12 µm and the distance between the edges of the electrodes in the second contact area falling within a range of 18 to 24 µm allows for obtaining an impedance change enabling identification of specific proteins. Attaching of the specific proteins for a virus by the antibodies is resulting in a step change of the impedance modulus, which can be measured by direct current or with a frequency in a range of 0,1 Hz to 50 kHz. The distances between the edges of the electrodes are crucial for a sensitivity and a specificity of tests, because the sizes of the proteins of the viruses differ. By keeping the required values of the distance between the edges of the electrodes and the heights of the electrodes described above it is possible to use different shapes of the electrodes.

Advantageously, as the substance comprising antibodies binding proteins of the first virus is used a substance attaching specific proteins of the viruses from a group of coronaviruses, and as the substance comprising antibodies binding proteins of the second virus is used a substance attaching specific proteins of the viruses from a group of influenza viruses. In a case when it is found that a threshold condition is fulfilled by the impedance between the common electrode and the first electrode, a detection of proteins from a group of coronaviruses is stated, whereas in a case when it is found that the impedance between the common electrode and the second electrode, a detection of proteins of influenza virus is stated.

Advantageously, as the substance comprising antibodies binding specific proteins from a group of coronaviruses, a substance attaching specific proteins of virus SARS-CoV-2 is used and in a case when it is found a threshold condition is fulfilled by the impedance between the common electrode and the first electrode, a detection of virus SARS-CoV-2 is stated.

Advantageously, the substance under test is the air exhaled by the patient.

A device for detection of proteins of viruses, according to the invention comprises a measurement chamber and a sensor, wherein the sensor is a sensor suitable for detection of proteins of viruses according to the invention, whose electrodes are placed in the measurement chamber, and the measurement chamber is provided with an inlet opening and an outlet opening wherein a mouthpiece is connected to the inlet opening, and between the mouthpiece and the inlet opening there is a filter.

The invention is based on an observation, that viruses can be identified by proteins specific for them and with a proper height of the electrodes and a distance between them, during an exposition on substances binding proteins specific for viruses, it comes to a situation, in which the proteins situated in a gap between the electrodes having a shape approximately of letter "Y" align themselves vertically, and they short-circuit the electrodes with their arms and the change of the impedance can be observed. If a gap between the electrodes is bigger than the size of the protein, no short-circuit occurs. If a height of the electrode is significantly bigger than the height of the protein, the short-cut happens relatively rarely. Also, the short-cut happens relatively rarely when the proteins of the virus are not bond with the substance corresponding to it, for example comprising proper antibodies. The set of heights of the electrodes and distances between them is selected specifically for distinguishing coronaviruses and influenza.

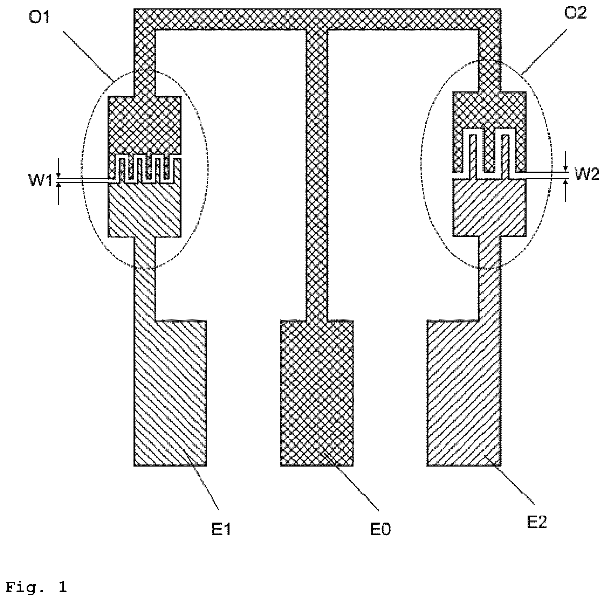

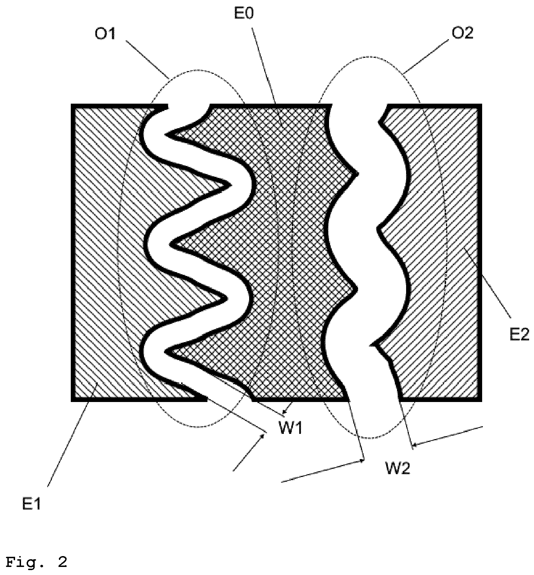

The embodiments of the invention are described below with reference to attached drawings, wherein Fig. 1 shows schematically the electrodes according to the embodiment of the invention, whereas Fig. 2 shows schematically the electrodes according to the alternative embodiment of the invention.

The biological sensor suitable for detection of proteins of viruses according to the invention comprises the electrodes connected to the impedance meter, as shown schematically in Fig. 1. The common electrode **E0** has two contact areas **O1** and **O2.** The electrodes are provided in a form of metalized areas on a common substrate. Depending on the parameters of a technological process, the electrodes can be of different heights. The height of the electrode above the substrate can change on a surface of the substrate. The thinner the electrodes, the more difficult it is to control their shape.

As shown in Fig. 1, in the contact areas **O1** and **O2**, the common electrode **E0**, the first electrode **E1** and the second electrode **E2** are provided with arranged alternately finger-like protrusions.

In the first contact area **O1**, the common electrode **E0** is placed in the neighborhood of the first electrode **E1**. In this area, the length **W1** between the edge of the common electrode **E0** and the first electrode **E1** is 10 µm. The height of the first electrode **E1** and the common electrode **E0** in the contact **O1** area is 150 nm. Also electrodes of smaller height can be used - it is important that the antibodies arranged substantially perpendicularly to the substrate have arms protruding above the metallization of the electrodes in the contact area.

In the first contact area **O1**, the common electrode **E0** and the first electrode **E1** are covered with a substance comprising antibodies attaching specific proteins of SARS-CoV-2 virus - commercially available Rabbit Anti-SARS-CoV-2 NP Polyclonal antibody, (CABT-CS024).

Finger-like protrusion located in the contact area **O1** - four of each of the common electrode **E0** and the first electrode **E1** are marked schematically in Fig.1. It is only a simplification allowing to illustrate the invention in more transparent way. In the embodiment, 20 finger-like protrusions arranged alternately are used, having a width of 5 µm and a length of 10 mm. Thanks to such configuration, the value of the impedance modulus between electrodes is of the order of tens of kiloohms.

In the second contact area **O2**, the common electrode **E0** is located in the neighborhood of the second electrode **E2**. In this area, the length **W2** between the edge of the common electrode **E0** and the second electrode **E2** is 21 µm. The height of the second electrode **E2** and the common electrode **E0** in the contact area **O2** is 450 nm. Also electrodes of lower height can be used - it is important for the antibodies arranged substantially perpendicularly to the substrate have their arms protruding above the metallization of the electrodes in the contact area.

In the second contact area **O2**, the common electrode **E0** and the second electrode **E2** are covered with a substance comprising antibodies attaching specific proteins of influenza viruses - commercially available Mouse anti influenza A H1 antibody, clone 58AB7-19-18 (OBT1562).

Finger-like protrusion - three of each of the common electrode **E0** and the second electrode **E2** are marked schematically in Fig.1. It is only a simplification allowing to illustrate the invention in more transparent way. In the embodiment, 20 finger-like protrusions arranged alternately are used, having a width of 15 µm and a length of 15 mm. Thanks to such configuration, the value of the impedance modulus between electrodes is of the order of tens of kiloohms.

Attaching of the specific proteins for the virus by the antibodies in the contact area **O1**, **O2** results in the short-circuit of the electrodes by proteins, and, as a consequence, the step change of the value of the impedance modulus measured by direct current or with a frequency in a range of 0,1 Hz to 50 kHz. In the experiments it was found that the changes of the impedance allowing for identification of specific proteins can be obtained by providing the length **W1** between the edges of the electrodes in the first contact area **O1** falling within a range of 8 to 12 µm and the length **W2** between the edges of electrodes in the second contact area **O2** falling within a range of 18 to 24 µm. Values of the length **W1** and **W2** are important, because they correspond to the sizes of attached proteins, which can come into contact with to electrodes at the same time. Because the sizes of the proteins of the viruses differ, the lengths **W1** and **W2** are crucial for a sensitivity and a specificity of tests.

The common electrode **E0** and the first electrode **E1** are connected to the bridge impedance meter. The common electrode **E0** and the second electrode **E2** are also connected to the bridge impedance meter. The same meter can be used and the first electrode **E1** and the second electrode **E2** can be connected to it through the switch layout providing them alternate electrical contact with the meter terminals.

Also other shapes of the electrodes can be used in the contact areas **O1** and **O2** - as shown schematically in Fig. 2. It is important to maintain required values of the lengths **W1** and **W2** and the heights of the electrodes pointed above.

Also more complex conditions concerning measured impedance, in particular based on the change of capacitance can be proposed.

In particular, the substance under test can be the air exhaled by the patient.

A device for detection of proteins of viruses, according to the invention comprises a measurement chamber and a sensor. The sensor is a sensor suitable for detection of proteins of viruses according to the invention, whose electrodes are placed in the measurement chamber, and the measurement chamber is provided with an inlet opening and an outlet opening wherein a mouthpiece is connected to the inlet opening, and between the mouthpiece and the inlet opening there is a filter for capturing large particles and condensate.

The invention provides a possibility of simultaneous determination of infection of SARS-CoV-2 and influenza virus on a basis of testing the patient-derived substance, in particular the exhaled air. Determination on a basis of one sample makes the process simpler and facilitates treatment, especially in a situation, when both viruses are present in population.

The embodiments of the invention described above should not be interpreted as limiting the idea of the invention to the pointed embodiments of the invention. For the person skilled in the art it is obvious, that embodiments can be subjected to many modifications, adaptations or equal realizations, which will not be too distant from the invention and its technical character and will not lead to degrading the technical effects obtained by them. In particular, the person skilled in the art routinely selects substances comprising antibodies binding proteins of the first virus and the second virus, respectively.

## Claims

1. A biological sensor for detection of proteins of viruses comprising electrodes connected to an impedance meter, **characterized in that** the sensor is provided with a common electrode **(E0),** a first electrode (**E1**), and a second electrode **(E2),** wherein
those electrodes **(E0, E1**, **E2**) are provided in a common substrate so that they have a first contact area **(O1)** covered with a first substance comprising antibodies binding proteins of a first virus, in which in the said first contact area **(O1),** a distance **(W1)** between an edge of the common electrode **(E0)** and an edge of the first electrode **(E1)** is falling within a range of 8 to 12 µm and a height of the electrodes is falling within a range of 50 to 150 nm, and those electrodes **(E0, E1**, **E2**) have a second contact area
**(O2)** covered with a second substance comprising antibodies binding proteins of the second substance, in which in the said second contact area **(O2),** a distance **(W2)** between the common electrode (**E0**) and the second electrode **(E2)** is falling within a range of 18 µm to 24 µm, whereas a height of electrodes above the substrate is falling within a range of 50 to 450 nm,
wherein
the common electrode **(E0)** and the first electrode **(E1)** are connected to the bridge impedance meter
and
the common electrode **(E0)** and the second electrode **(E2)** are connected to the bridge impedance meter.

2. Biological sensor for detection of proteins of viruses according to claim 1, **characterized in that** the common electrode **(E0),** the first electrode **(E1)** and the second electrode **(E2)** have finger-like protrusions in the contact areas **(O1, O2).**

3. Biological sensor for detection of proteins of viruses according to claim 1 or 2, **characterized in that** the substance comprising antibodies binding proteins of the first virus is a substance attaching specific proteins of viruses form a group of coronaviruses.

4. Biological sensor for detection of proteins of viruses according to claim 3, **characterized in that** the substance comprising antibodies binding proteins of the first virus is a substance attaching specific proteins of virus SARS-CoV-2.

5. Biological sensor for detection of proteins of viruses according to claim 1 or 2, **characterized in that** the substance comprising antibodies binding proteins of the second virus is a substance attaching specific proteins of viruses of influenza.

6. Biological sensor for detection of proteins of viruses according to claim from 1 to 5, **characterized in that** the finger-like protrusions in the contact areas have a width falling within a range of 5 µm to 15 µm and a length falling within a range of 3 to 15 mm.

7. A device for detection of proteins of viruses, comprising a measurement chamber and a sensor, **characterized in that** the sensor is a sensor suitable for detection of proteins of viruses, as described in any of claims from 1 to 6, whose electrodes are placed in the measurement chamber, and the measurement chamber is provided with an inlet opening and an outlet opening wherein a mouthpiece is connected to the inlet opening, and between the mouthpiece and the inlet opening there is a filter.
